Europäisches Patentamt

European Patent Office

Office européen des brevets

(19)

(11) Publication number: **0 416 343 A2**

# EUROPEAN PATENT APPLICATION

(12)

(21) Application number: 90115690.1

(22) Date of filing: **16.08.90**

(51) Int. Cl.5: **C12P 19/04**, //C12R1:725

(30) Priority: **04.09.89 IT 2160689**

(43) Date of publication of application:
**13.03.91 Bulletin 91/11**

(84) Designated Contracting States:
**AT BE CH DE DK ES FR GB GR IT LI LU NL SE**

(71) Applicant: **CONSIGLIO NAZIONALE DELLE RICERCHE**
**Piazzale Aldo Moro, 7**
**I-00195 Roma(IT)**

Applicant: **ISTITUTO SUPERIORE DI SANITA**
**Viale Regina Elena, 299**
**I-00101 Roma(IT)**

(72) Inventor: **Cassone, Antonio**
**Via Meropia, 112**
**I-00147 Rome(IT)**
Inventor: **Bistoni, Francesco**
**Via Briganti, 85**
**I-06100 Perugia(IT)**
Inventor: **Marconi, Pier Francesco**
**Via Briglia di Braccio, 8**
**I-06100 Perugia(IT)**

(74) Representative: **Gervasi, Gemma et al**
**NOTARBARTOLO & GERVASI Srl Viale**
**Bianca Maria 33**
**I-20122 Milan(IT)**

(54) **Process for preparing a glucane-containing product starting from candida albicans BMM-12.**

(57) There is described a process for preparing a product containing easily purifiable glucane starting from Candida albicans ; the product has the physical appearance of microscopic glucane bodies.

EP 0 416 343 A2

## PROCESS FOR PREPARING A GLUCANE-CONTAINING PRODUCT STARTING FROM CANDIDA ALBICANS BMM-12

### FIELD OF THE INVENTION

This invention relates to a process for preparing a product containing easily purifiable glucane starting from selected stocks of Candida albicans identified and numbered as BMM-1, BMM-2 etc. up to BMM-12, the product so obtained and the Candida albicans stock necessary to carry out the abovesaid preparation process.

The micro-organism utilized in the present invention, designated as BMM-12, was deposited on the 4th of August 1989 with the American Type Culture Collections, according to the Budapest Treaty on the patent procedures. This institute is recognized according to the Budapest Treaty on the patent procedures. The American Type Culture Collections Institute is recognized for the deposit of the microorganisms according to rule 28EPC. The deposit number of the stock utilized in this application is indicated as:
ATCC 20955.

### PRIOR ART

Glucane is a polysaccharide which is present, in nature, for example in the cellular parietes of fungic microorganisms, in particular of yeasts.

Glucanes of different origin, for example from different microorganisms, differ from one another and, furthermore, different processes and treatments which said microorganisms are subjected to, including the cultivation and maintenance modalities, lead to the obtainment of different end products.

Such differences can be noticed both on the tridimensional structure of the polysaccharide chain or of the chemical bonds among the glucopyranosidic units of such chain, and on the biological activity of the glucanes as well as on the presence of substances other than glucane in the raw product, with consequent greater or lesser easiness of purification.

Glucanes from Saccharomyces cerevisiae or from Lentinus edodes , both having a branched structure with predominance of $\beta$-1,3-glucopyranosidic bonds, are known.

Said glucanes are particularly studied because of their antitumoral and antibacterial activity (Int. J: Cancer 24, 773-779 (1979); Int. J: Immunopharmac. Vol. 7, No. 5, 747-751 (1985)). Furthermore, they exhibit an immunomodulator effect both in vivo and in vitro (Rev. Microbiol. Vol. 15 , 87-96, 1987) and exert a radioprotectice action (Meth. and Find.

Exptl. Pharmacol. Vol. 8, No. 3, 151-155 (1986)). Glucanes have been produced also starting from Candida albicans and the immunomodulator effect thereof has been studied (J. Gen. Microbiol. Vol. 134, 1265-74 (1988)).

### TECHNICAL PROBLEM

The glucanes known so far, however, are not fully suited to be used for biological treatments on humans.

In fact, they are obtained by means of processes which do not remove polluting substances such as, for example, proteins, lipides or residues of nucleic acids, which are toxic agents and/or sensibilizers for humans and which, by consequence, render such glucanes not utilizable for prolonged or repeated treatments on human beings.

An object of the present invention is therefore a process for preparing a product defined as "parietal glucanic bodies" containing alkalo/acid-insoluble glucane for at least 90%, by making use of the Candida albicans BMM-12 stock.

By means of this process it is possible to obtain a rough glucane, which permits to successively obtain a pure glucane not affected by the abovesaid drawbacks and exhibiting a superior activity and fewer undersirable effects in the biological treatments on animals and humans.

### DETAILED DESCRIPTION OF THE INVENTION

Whenever used in the present invention, the term "glucane" means a composition of glucane (at least 90%) and chitin (at least 3%). The product according to the invention is obtained from fungus Candida albicans , stock BMM-12 (ATCC 20955). The fungus is maintained in culture on a Sabouraud solid soil (glucose: 20 g/100 ml; peptone: 10 g/100 ml; $H_2O$: 100 ml, pH adjusted at 6.5, agar: 1.5 g/100 ml), where it grows till reaching a confluent patina in 1-2 days at 28°C; then it is kept at room temperature or at 4°C for all the necessary time prior to extraction and preparation of the "parietal glucanic bodies". To this purpose, the fungus is then made to grow, till reaching the stationary stage, in a Winge soil (glucose: 0.3% by weight, and yeast extract: 0.1% by weight in distilled water) at 28°C in a shaker (stirring at about 50 rpm).

The cellular mass is collected after centrifugation, which is generally carried out at 2-3000 rpm for 15 minutes, and then it is suspended in an aqueous solution of a citrate buffer at a pH of 5.0, whereafter it is placed into an autoclave at 121°C for 3 hours. After having discharded the overfloating product, the cells were subjected three times to the following cycle:

a) dispersion in a NaOH solution at 1% by weight at 100°C for 24 hours and subsequent washings with sterile and apyrogenic water till neutralization;

b) dispersion in a 0.5 solution of $CH_3COOH$ at 80°C for 24 hours and subsequent washings with water until neutralization.

After the last washing, the suspension is centrifuged at 4-5000 rpm for 30 minutes and the resulting pellet is defined as "parietal glucanic bodies". The product yield is of about 13% referred to the initial cells and exhibits a glucane content not lower than 90% by weight. The product has a granular, whitish or colorless appearance and, resuspended in water or in physiological solution it spontaneously precipitates.

An ultrastructural analysis under an electron microscope reveals that the product consists of more or less regular granular bodies having a thickness of about 200 nm and a total size (diameter) around 3-6 micron.

## Claims

1. A process for preparing a product containing at least 90% of glucane, characterized by the use of Candida albicans stock BMM-12 (ATCC 20955).

2. A microorganism of genus Candida having the capability of producing a product containing (for at least 90%) easily purifiable glucane.

3. Candida albicans BMM-12 (ATCC 20955) for preparing, according to the process of claim 1, a product containing at least 90% of glucane.

4. The product containing at least 90% of glucane, obtained through the process as claimed in claim 1.

5. The product according to claim 1, wherein glucane represents about 90%, the balance being substantially chitin.